# EUROPEAN PATENT APPLICATION

(11) **EP 3 673 957 A1**
(43) Date of publication of application: **01.07.2020**
(21) Application number: 17927390.9
(22) Date of filing: 12.10.2017
(51) Int. Cl.: A61N 7/02, A61N 7/00

(54) **FOCUSED ULTRASOUND DEVICE**

(30) Priority: 28.09.2017 KR 20170126315
(71) Applicant: Cho, Sung-Chan, Gunpo-si, Gyeonggi-do 15851 (KR)
(72) Inventor: Cho, Sung-Chan, Gunpo-si, Gyeonggi-do 15851 (KR)
(74) Representative: Manitz Finsterwald Patent- und Rechtsanwaltspartnerschaft mbB
(86) International application number: PCT/KR2017/011229
(87) International publication number: WO 2019/066117

(57) **Abstract**

A focused ultrasound device includes an ultrasound transducer having a piezoelectric element for generating ultrasound waves, a housing in which the ultrasound transducer is installed, and a camera module configured to photograph a surface of a human body onto which the ultrasound waves generated by the ultrasound transducer are irradiated.

## Description

### [Technical Field]

The present invention relates to a focused ultrasound device configured to generate and focus ultrasound.

### [Background Art]

A focused ultrasound device is a device that generates and focuses ultrasound and is widely used for a therapeutic effect. A therapeutic effect may be obtained by irradiating the focused ultrasound generated by a focused ultrasound device to a human body, and the ultrasound generated from the piezoelectric element may be focused inside the human body and a desired therapeutic effect may be achieved by heat generated at the focused point.

In general, a focused ultrasound device includes a piezoelectric element for generating ultrasound waves, and a housing surrounding the piezoelectric element, and a space in the housing in which the piezoelectric element is disposed is filled with ultrasound transmitting medium (for example, water) for transmission of ultrasound waves. At this time, a portion which contacts a human body and through which the ultrasound wave generated by the piezoelectric element passes is formed as a thin film through which the ultrasound wave can pass.

In order to irradiate ultrasound to the human body using such a focused ultrasound device, the ultrasound should be accurately irradiated at a desired treatment position, and the housing of the focused ultrasound device blocks an area where the ultrasound is transmitted so that a treatment position should be adjusted by an experience or a feeling of an operator in a prior art. As a result, there is a problem that it is difficult to precisely irradiate ultrasound waves at the correct position.

In order to precisely adjust the treatment position, a focused ultrasound device equipped with a diagnostic ultrasound imaging probe has been introduced, but the diagnostic ultrasound imaging probe cannot suggest the exact treatment position because it shows a cross-sectional state of a tissue as ultrasound images. In particular, if the location of the treatment is very sensitive (position under an eye, area near a certain point, etc.) or if a focused ultrasound device should be inserted into a body cavity (e.g., throat, vagina, etc.) for treatment so that it cannot be seen to eyes, the correct location of the treatment may not be precisely identified.

### [Prior documents]

- Korea patent publication No. 10-2016-0080892 (publication date: July 08, 2016)
- Korea patent publication No. 10-2017-0055734 (publication date: May 22, 2017)

### [Detailed Description of the Invention]

### [Technical Problem]

The problem to be solved by the present invention is to provide a focused ultrasound device for visually indicating a focused position of ultrasound while performing focused ultrasound treatment so that ultrasound can be irradiated to a desired precise position.

### [Technical Solution]

A focused ultrasound device according to an embodiment of the present invention includes: an ultrasound transducer having a piezoelectric element for generating ultrasound waves; a housing in which the ultrasound transducer is installed; and a camera module configured to photograph a surface of a human body onto which the ultrasound waves generated by the ultrasound transducer are irradiated.

The camera module may be disposed inside the housing or on the housing.

The camera module may be disposed within the piezoelectric element.

The camera module may be disposed at a center of the piezoelectric element.

The camera module may be disposed outside the piezoelectric element.

In another embodiment, a focused ultrasound device may further include a lighting module for illuminating an image photographing region of the camera module.

The lighting module may be disposed inside the housing or on the housing.

The lighting module may be disposed near a periphery of the camera module.

The lighting module may be disposed outside an outer periphery of the piezoelectric element.

The ultrasound transducer may be provided with a through hole into which the camera module is installed. The focused ultrasound device may further include a tube member that is inserted into the through hole in a state of sealing the through hole, and the camera module may be disposed in the tube member.

The focused ultrasound device may further include a lighting module for illuminating an image photographing region of the camera module, and the lighting module may be disposed within the tube member together with the camera module.

A front portion of the tube member may be formed of a transparent material and a rear end thereof may be formed to be sealed.

The housing may have an opening portion through which the ultrasound passes and an ultrasound transmitting film covering the opening portion. The focused ultrasound device may further include a lighting module that is installed within the housing and is configured to illuminate an image photographing region of the camera module. The camera module may be arranged to photograph through the ultrasound transmitting film and the lighting module is arranged to illuminate through the ultrasound transmitting film, and the ultrasound transmitting film may be formed of a transparent material or a partially transparent material to enable a photographing by the cameral module and an illumination by the lighting module.

The focused ultrasound device may further include a support that is configured to be coupled to and decoupled from the housing, and the camera module may be mounted to the support.

The housing may be provided with an insertion hole for an insertion of the support, and the support may be inserted into the insertion hole.

The camera module may be provided in plurality.

The focused ultrasound device may further include a lighting module configured to illuminate a human body surface onto which the ultrasound is irradiated, and the lighting module may be mounted to the support together with the camera module.

The lighting module may be provided in plurality.

The cameral module may be installed to the housing, and the ultrasound transducer may be formed as a part of an ultrasound transducer module that is configured to be separated from and be coupled to the housing.

The ultrasound transducer module may include: a support structure that supports the ultrasound transducer and is configured to be able to be coupled to the housing; a hollow pipe that is installed to penetrate the support structure and the ultrasound transducer; and a transparent cover that is coupled to a front end of the hollow pipe. The camera module may be configured such that a front end portion of the camera module is inserted into the hollow pipe in a state that the ultrasound transducer module is coupled to the housing.

The ultrasound transducer module may include: a support structure that supports the ultrasound transducer and is configured to be able to be coupled to the housing; a through hole that penetrates the support structure and the ultrasound transducer; and a transparent cover that is coupled to the through hole. The camera module may be inserted into the through hole in a direction toward the transparent cover in a state that the ultrasound transducer module is coupled to the housing.

The ultrasound transducer module may include: a support structure that supports the ultrasound transducer and is configured to be able to be coupled to the housing; a hollow pipe that is installed to penetrate the support structure and the ultrasound transducer; and a transparent cover that is coupled to a front end of the hollow pipe. The camera module may be installed in the hollow pipe toward the transparent cover in a state that the ultrasound transducer module is coupled to the housing.

In another embodiment, the focused ultrasound device may further include a lighting module that is configured to illuminate a human body surface on which the ultrasound is irradiated, and the lighting module may be inserted into the hollow pipe or the through hole together with the camera module.

Meanwhile, a focused ultrasound device according to another embodiment of the present invention includes: an ultrasound transducer having a piezoelectric element for generating ultrasound waves; a housing in which the ultrasound transducer is installed; a camera module configured to photograph a surface of a human body onto which the ultrasound waves generated by the ultrasound transducer are irradiated; and an image display module displaying an image of a human body surface photographed by the camera module. The image display module is configured to display ultrasound irradiation information on the image of the surface of the human body photographed by the camera module.

The ultrasound irradiation information may include at least one of an ultrasound transmitting region penetrating a human body surface, a position of an ultrasound focus formed within a human tissue, a depth from a human body surface to the ultrasound focus, an entire region on which the ultrasound irradiates while the ultrasound transducer moves, an entire region on which the ultrasound focus is located within the human tissue while the ultrasound transducer moves, an overall length of a trajectory of the ultrasound focus, and an interval between the ultrasound foci.

The housing may include an opening portion through which the ultrasound passes and an ultrasound transmitting film that covers the opening portion, and the ultrasound transmitting region may be displayed to respond to a region in which the ultrasound generated by the ultrasound transducer passes through the ultrasound transmitting film.

The ultrasound transducer may be formed as a plurality of replaceable cartridges, and the ultrasound irradiation information may be obtained based on an ultrasound focusing shape of the ultrasound transducer.

The ultrasound transducer may be installed to be movable, rotatable and tiltable, and the image display module may be configured to variably display the ultrasound irradiation information in accordance with the ultrasound irradiation information that changes in accordance with movement, rotation or tilting of the ultrasound transducer.

### [Advantageous Effects]

According to the present invention, since an image photographed by a camera module is displayed on an image display module, it is possible to check images of a surface of a human tissue onto which ultrasound is irradiated during the ultrasound treatment so that ultrasound can be irradiated to a desired precise point.

### [Brief Description of Drawings]

FIG. 1 is a cross-sectional view showing an example of a focused ultrasound device according to an embodiment of the present invention.
FIG. 2 is a view showing an arrangement of a camera module and an illumination module of a focused ultrasound device according to an embodiment of the present invention.
FIG. 3 is a view showing an arrangement of a camera module and an illumination module of a focused ultrasound device according to another embodiment of the present invention.
FIG. 4 is a view showing an arrangement of a camera module and an illumination module of a focused ultrasound device according to yet another embodiment of the present invention.
FIG. 5 is a schematic cross-sectional view of a focused ultrasound device according to another embodiment of the present invention.
FIG. 6 is another cross-sectional view of a focused ultrasound device of FIG. 5.
FIG. 7 is a bottom view of a focused ultrasound device of FIG. 5.
FIG. 8 is a schematic cross-sectional view of a focused ultrasound device according to another embodiment of the present invention.
FIG. 9 is a bottom view of a focused ultrasound device of FIG. 8.
FIG. 10 is a view illustrating a state in which an ultrasound transducer module is separated from a focused ultrasound device of FIG. 8.
FIG. 11 exemplarily shows a display of an ultrasound focusing position on a photographed image by a display module of a focused ultrasound device according to an exemplary embodiment of the present invention.
FIG. 12 shows an example of displaying irradiation information of ultrasound on an image photographed by a camera module of a focused ultrasound device according to an embodiment of the present invention.

### [Detailed Description of the Embodiments]

Hereinafter, an embodiment of the present invention will be described in detail with reference to the accompanying drawings.

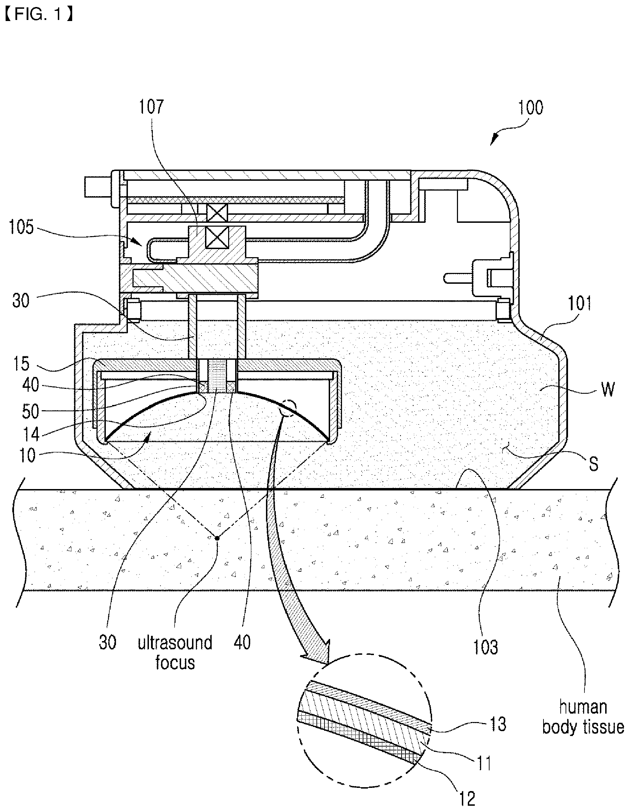

A focused ultrasound device according to an embodiment of the present invention is a device for generating focused ultrasound that can be used for therapeutic purposes. Referring to FIG. 1, a focused ultrasound device 100 may include an ultrasound transducer 10 that generates ultrasound, and the ultrasound transducer 10 may be installed in a housing 101.

The housing 101 may form an installation space S in which the ultrasound transducer 10 is disposed, and the installation space S may be filled with ultrasound transmission medium (e.g., water) W. Ultrasound waves generated by the ultrasound transducer 10 may proceed through the ultrasound transmission medium W and may pass through a surface of a human body in close contact outside the housing 101 to be focused therein. To this end, the housing 101 may be configured to have an open portion through which ultrasound wave proceeds, and an ultrasound wave transmitting film 103 through which the ultrasound wave may pass may be provided in the open portion. The ultrasound transmitting film 103 may be formed in the form of a thin film of a synthetic resin material to seal the ultrasound transmission medium W and to allow ultrasound waves to pass therethrough. At this time, as shown schematically in FIG. 1, the ultrasound treatment is performed in a state where the ultrasound transmitting film 103 is in close contact with a surface of a human tissue. Although not specifically shown in the drawings, the housing 101 may be formed so that an operator can hold it by hand, and may also be formed in a shape in which the tip portion thereof may be inserted into a body such as a throat or a vagina.

The ultrasound transducer 10 may be installed to be movable within the housing 101, and a driving unit 105 for moving the ultrasound transducer 10 may be provided for this purpose. Although not shown in the drawings, the driving unit 105 may include a motor for providing a driving force. In this case, the ultrasound transducer 10 may include a support member 15 that serves as a support, and the support member 15 may be connected to the driving unit 105 through a connection member 30. For example, the ultrasound transducer 10 may be installed in the housing 101 to be movable in at least one of a transverse direction (left and right direction in FIG. 1) and a vertical direction (up and down direction in FIG. 1), or to be tiltable (left and right rotation in FIG. 1). For example, the connecting member 30 and the ultrasound transducer 10 connected thereto can be configured to be movable in the transverse direction by moving a moving member 107 of the drive unit 105, and also the connecting member 30 may be configured to be movable in the longitudinal direction. In addition, the connecting member 30 may be configured to be tiltable for the tilting behavior of the ultrasound transducer 10. The moving structure of the ultrasound transducer 10 can be easily configured by those skilled in the art by a motor, a linear motor, a gear, various power transmission members and the like, so a detailed description thereof will be omitted.

When the ultrasound transducer 10 moves in the transverse direction, the ultrasound focus formed inside the human tissue is moved in the transverse direction in FIG. 1 with a substantially constant depth from the surface of the human tissue, and on the other hand when the ultrasound transducer 10 moves in a vertical direction, the ultrasound focus formed inside the human tissue is moved in the vertical direction in FIG. 1 while a depth of the focus from a surface of the human tissue changes. In addition, when the ultrasound transducer 10 is tilted, the ultrasound focus formed inside the human tissue moves in a transverse direction and at the same time the depth thereof also changes. On the other hand, although not shown in the figure, the ultrasound transducer may be installed to be able to undergo a rotational or tilting behavior as well as a linear movement.

Meanwhile, although the structure in which the ultrasound transducer 10 is movable is illustrated in the drawing, in another embodiment of the present invention, the ultrasound transducer may be installed to be fixed at a predetermined position.

On the other hand, the ultrasound transducer 10 and the housing 101 supporting it may be configured in the form of a plurality of cartridges that can be replaced. For example, each cartridge may respectively include a housing including an ultrasound transducer having different ultrasound focusing characteristics, and a focused ultrasound treatment may be performed using a housing in which an appropriate ultrasound transducer cartridge is coupled as necessary.

The ultrasound transducer 10 may include a piezoelectric element 11 and first and second electrodes 12 and 13 formed on both surfaces of the piezoelectric element 11, respectively. As shown in the drawing, the piezoelectric element 11 and the first and second electrodes 12 and 13 formed on both surfaces thereof may be fixed to the support member 15. The piezoelectric element 11 may be formed of various materials capable of converting an electrical signal into mechanical vibration, such as a ceramic or a composite piezoelectric material, and the first electrode 12 and the second electrode 13 may be formed of a metallic material having a good electrical conductivity. Referring to FIG. 1, the first and second electrodes 12 and 13 may be formed on both surfaces of the piezoelectric element 11, respectively. Although not shown in the drawings, the first and second electrodes 12 and 13 may be connected to a pulse power generator (not shown) by an electrical cable. The pulsed power generated by the pulse power generator is applied to the first and second electrodes 12 and 13 via a cable, thereby causing ultrasound vibration of the piezoelectric element 11 to generate ultrasound waves.

The piezoelectric element 11 may be formed in a parabolic curved surface, a spherical surface, a cylindrical curved shape, or the like so as to focus the ultrasound wave at a point or an area spaced apart from the front by a predetermined distance. That is, the shape of the piezoelectric element 11 is not limited to the shape shown in the drawings and may be variously changed as necessary.

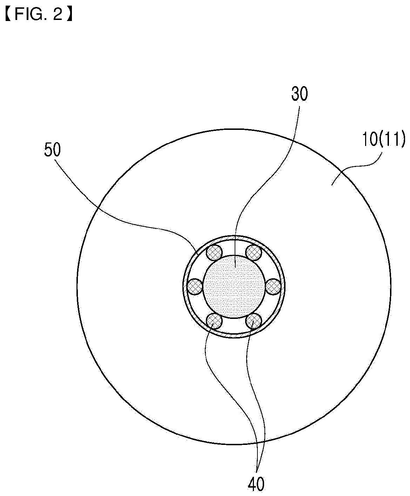

Meanwhile, referring to FIGs. 1 and 2, the focused ultrasound device 100 according to an embodiment of the present invention includes a camera module 30. The camera module 30 is configured to photograph (photographing downwardly in FIG. 1) the surface of the human tissue onto which the ultrasound generated by the ultrasound transducer 10 is irradiated. For example, the camera module 30 may be a digital camera or an imaging device (CCD, CMOS, etc.) that is capable of photographing images. In addition, the camera module 30 may include various types of camera lenses according to a photographing range.

In addition, since the camera module 30 may be located in the ultrasound transmission medium, the camera module 30 may be configured in its own sealed structure or disposed in a sealed structure.

According to an embodiment of the present invention, the camera module 30 may be disposed in the tube member 50 installed in the ultrasound transducer 10. For example, a through hole 14 may be formed in the ultrasound transducer 10, and the tube member 50 may be inserted into and installed in the through hole 14. In this case, the tube member 50 is installed to seal the through hole 14 so that the front and rear surfaces of the ultrasound transducer 10 are not electrically connected by the ultrasound transmission medium W. A space may be formed in the tube member 50, and the camera module 30 may be disposed in the space within the tube member 50. The front portion of the tube member 50 may be formed of a transparent material and the rear portion may be formed of a sealed structure. Thereby, the structure for waterproofing the camera module 30 and the lighting module 40 need not be provided separately.

Further, referring to FIGs. 1 and 2, the focused ultrasound device 100 may include a lighting module 40 configured to illuminate a photographing area of the camera module 30. The lighting module 40 may be implemented as any lamp capable of emitting light, for example, as an LED lamp. Illumination is achieved by the lighting module 40. Since the focused ultrasound device comes in close contact with the surface of the human body during the actual treatment, a separate lighting module is required because the light is blocked at the area where the ultrasound is irradiated, and in particular, in order to ensure the photographing by the camera module 30 in the space inside the human body where no light exists the lighting module 40 is required.

In this case, referring to FIG. 2, the lighting module 40 may be disposed inside the tube member 50 together with the camera module 30. In this case, the tube member 50 may be formed of transparent or low light reduction material to enable illumination and photographing an image.

In case that the camera module 30 and the lighting module 40 are disposed inside the tube member 50, if the front side of the tube member 50 is blocked by a transparent member and the rear side thereof is sealed, there is an advantage of preventing the ultrasound transmission medium inside the housing 101 from invading the camera module 30 and the lighting module 40 without a separate sealing structure for the camera module 30 and the lighting module 40.

In another embodiment of the present invention, the lighting module may be integrated with the camera module.

FIG. 2 is a view(that is, viewed downward from FIG. 1) showing an arrangement of the piezoelectric element 11, the camera module 30, and the illumination module 40 That is, according to an embodiment of the present invention, the camera module 30 may be disposed in the center of the piezoelectric element 11, and the illumination module 40 may be disposed adjacent to the peripheral edge of the camera module 30. Due to this arrangement, image capturing can be performed from the center of the ultrasound wave generated by the piezoelectric element 11, and the effective illumination of the region to be photographed is possible.

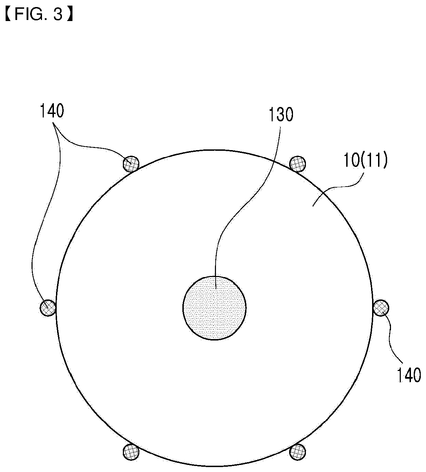

On the other hand, FIG. 3 shows the arrangement of the camera module 130 and the lighting module 140 according to another embodiment of the present invention. The camera module 130 is disposed at the center of the piezoelectric element 11, and the illumination module 140 is disposed outside the peripheral edge of the piezoelectric element 11. Meanwhile, in yet another embodiment, the camera module may be located at a point away from the center of the piezoelectric element.

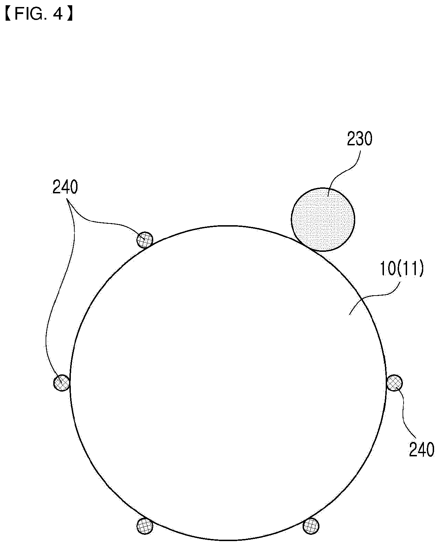

On the other hand, FIG. 4 shows the arrangement of the camera module 230 and the lighting module 240 according to yet another embodiment of the present invention. In this embodiment, the camera module 230 is disposed outside the piezoelectric element 11, and the lighting module 140 is also disposed outside the peripheral edge of the piezoelectric element 11.

In other embodiments of the present invention, the positions of the camera module and the lighting module may be variously changed. In one example, the camera module may be disposed outside the piezoelectric element and the lighting module may be disposed at the center of the piezoelectric element. In another example, the camera module may be disposed within the piezoelectric element at the center or a position away from the center, and the lighting module may be located outside the piezoelectric element and within the housing. In another example, the camera module and the lighting module may be disposed inside the housing without being disposed in the piezoelectric element. In another example, the camera module and the lighting module may be disposed outside the housing.

The ultrasound transmitting film 103 provided in the opening of the housing 101 through which ultrasound passes may be formed of transparent material or low light-absorbing material for lighting and photographing.

Meanwhile, the camera module 30 and the lighting module 40 described above may be provided as one or a plurality. When provided with a plurality, the photographing and lighting efficiency can be enhanced. In addition, the position of the camera module 30 and the illumination module 40 is not limited to those described above and may be disposed at any point within the housing 101 or on the housing 101.

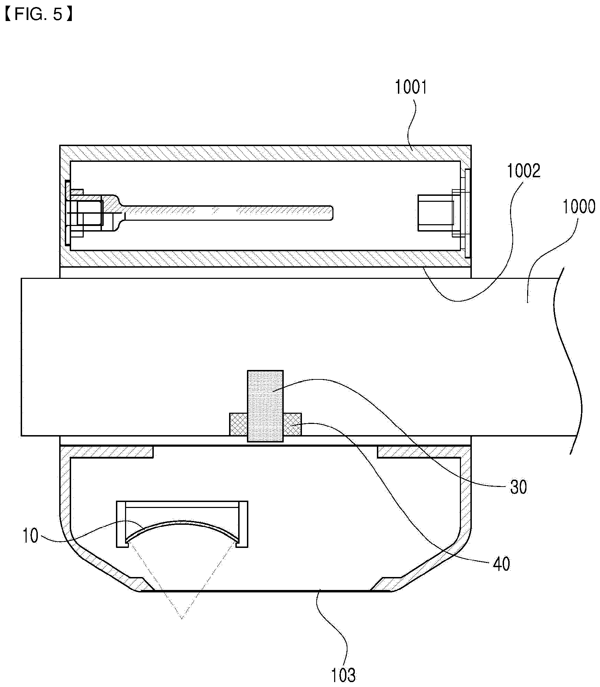

Hereinafter, the mounting structure of the camera module and the lighting module according to another embodiment of the present invention will be described with reference to FIGS. 5 to 7.

According to this embodiment, the camera module 30 is mounted on a separate support 1000. The support 1000 is a member separate from the housing 1001 and has a structure that can be separated from the housing 1001. As shown in FIGs. 5 to 7, an insertion hole 1002 is provided in the housing 1001, and the support 1000 is configured to be inserted into the insertion hole 1002. In this case, a lighting module 40 for illumination may be installed on the support 1000 together with the camera module 30. Meanwhile, in another embodiment of the present invention, the lighting module and the camera module may be installed on separate supports respectively and may be configured to be separately coupled to the housing via respective supports.

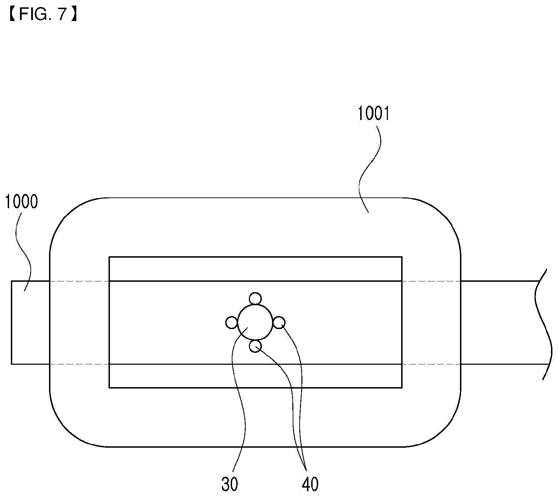

Referring to FIGs. 5 to 7, if the support 1000 is coupled to the housing 1001, the camera module 30 and the lighting module 40 may be disposed to face the treatment region that is required to be photographed. That is, as shown in the drawing, the camera module 30 is arranged to photograph in the direction of the propagation of the ultrasound generated by the ultrasound transducer 10, and the lighting module 40 is arranged to be able to illuminate the photographing region. To this end, as shown in FIG. 7, the housing 1001 may have an opening formed at a portion facing the camera module 30 and the lighting module 40 and photographing and lighting may be performed through the opening. In this case, the members positioned where the camera module 30 and the lighting module 40 face may be formed of a transparent material to allow photographing and lighting, and the camera module 30 and the lighting module 40 may photograph and illuminate the body surface through which ultrasound is irradiated through the transparent members, the ultrasound transmitting medium and the ultrasound transmitting film 103.

In the present embodiment, since the camera module 30 and the lighting module 40 are not mounted to the housing 1001 but are installed on the external support 1000 that can be separated from and combined with the housing 1001, the camera module 30 and the lighting module 40 do not need to be replaced together even when the ultrasound transducer 10 needs to be replaced due to the breakage of the ultrasound transducer 10 or failure or defect of the housing. This can reduce maintenance and repair costs.

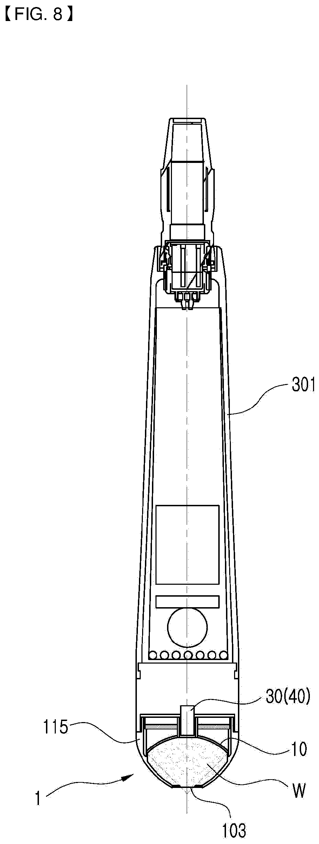

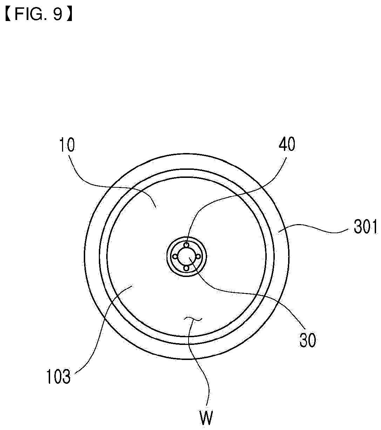

FIGs. 8 to 10 show another embodiment of the present invention. Referring to FIGs. 8 to 10, an ultrasound transducer module 1 including the ultrasound transducer 10 is configured to be separated from the housing 301 and replaceable. For example, the ultrasound transducer module 1 includes a support structure 115 that is configured to secure the ultrasound transducer 10 and on the other hand to be fastened to and detached from the housing 301. A portion of the housing 301 through which the ultrasound generated in the ultrasound transducer 10 passes is formed as an opening, and the ultrasound transmitting film 103 is disposed there.

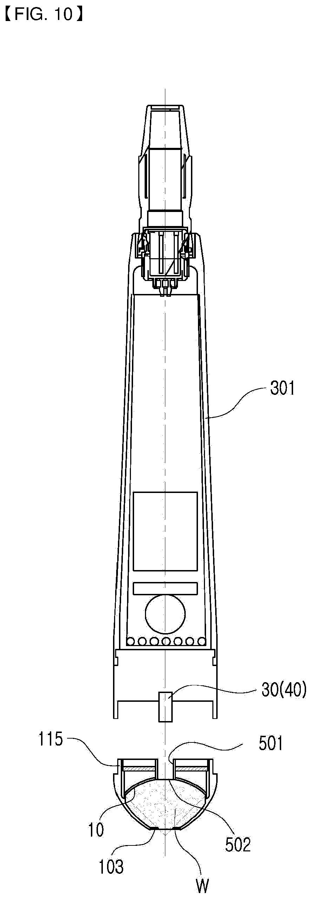

As shown in FIGs. 8 and 10, the camera module 30 may be mounted to the housing 301, and it is configured such that the camera module 30 can photograph the treatment region through the ultrasound transmitting film 103 when the ultrasound transducer module 1 is coupled to the housing 301. To this end, the ultrasound transducer module 1 may include a hollow pipe 115 that penetrates the support structure 115 and the ultrasound transducer 10 and a transparent cover 502 that is coupled to a frontal end of the hollow pipe 115 to ensure the sealing and the light transmission. As shown in FIG. 8, the front end of the camera module 30 may be installed to protrude, and when the ultrasound transducer module 1 is coupled to the housing 301, the protruded front end of the camera module 30 may be inserted in the hollow pipe 501 in the direction of the transparent cover 502. In this case, the lighting module 40 may be disposed together with the camera module 30.

Although not shown in the drawings, instead of the hollow pipe 501, a simple through hole that penetrates through the support structure and the ultrasound transducer, and the camera module 30 may be inserted therein.

Thus, since the camera module 30 is installed in the housing 301 and the ultrasound transducer module 1 is configured to be able to be replaced, the camera module 30 can be remained when the replacement of the ultrasound transducer module 1 is required, so maintenance and repairing costs can be reduced.

The focused ultrasound device 100 according to an exemplary embodiment of the present invention may further include an image display module 60 displaying an image of the human body surface photographed by the camera module 30. The image display module 60 may be implemented as any image display device capable of displaying an image, for example, as a liquid crystal display device.

The image display module 60 receives an image signal from the camera module 30 and displays it as an image. The image display module 60 is configured to display ultrasound irradiation information on the photographed human body surface image. By additionally displaying the ultrasound irradiation information on the photographed body surface image by the image display module 60, an operator can intuitively observe the state of the ultrasound irradiation from the displayed image.

According to an embodiment of the present invention, when the ultrasound transducer is installed to be movable, rotatable or tiltable, the ultrasound transducer information may be variably displayed according to the irradiation characteristics of the ultrasound wave that is varied according to the movement, rotation or tilting of the ultrasound transducer.

Specifically, the ultrasound irradiation information may include at least one of an ultrasound transmitting region penetrating the human body surface, a position of an ultrasound focus formed within the human tissue, a depth from the human body surface to the ultrasound focus, an entire region on which ultrasound irradiates while an ultrasound transducer moves, an entire region on which ultrasound focus is located within the human tissue while the ultrasound transducer moves, an overall length of a trajectory of the ultrasound focus, and an interval between the ultrasound foci. The ultrasound transmitting region penetrating the human body surface can be estimated to correspond to the transmitting region of ultrasound waves passing through the ultrasound transmitting film 103 because the ultrasound treatment is performed in a state in which the ultrasound transmitting film 103 is in close contact with the human body surface, and the transmitting region of ultrasound waves passing through the ultrasound transmitting film 103 may be theoretically or experimentally determined through the geometrical characteristics of the ultrasound transducer 10, the distance between the ultrasound transducer 10 and the ultrasound transmitting film 103, and the like. The ultrasound focus refers to a focus on which ultrasound waves passing through the surface of the human tissue are focused within the human tissue. The ultrasound focal position may be calculated based on the photographing area of the camera module 30, the geometrical features of the ultrasound transducer 10, the distance between the ultrasound transducer 10 and the ultrasound transmitting film 103, and the like. For example, the focal length may be calculated by the geometrical characteristics of the ultrasound transducer 10, and the ultrasound focal depth may be obtained by subtracting the distance between the ultrasound transducer 10 and the ultrasound transmitting film 103 from the calculated focal length.

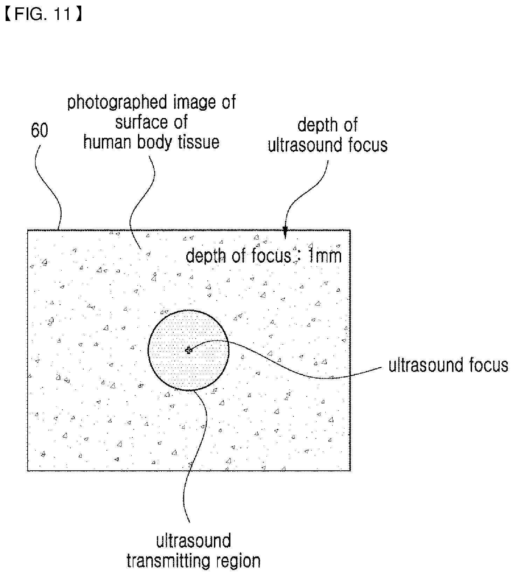

As exemplarily shown in FIG. 11, an ultrasound transmitting region and an ultrasound focus may be displayed on a photographed image of a human body, and an ultrasound focal depth may also be separately displayed in a numerical form. By the ultrasound irradiation information, an operator can check the ultrasound region passing through the human body surface on the photographed image of a human body surface, and can intuitively check the position of the ultrasound focus formed within the human tissue and the depth thereof from the human body surface.

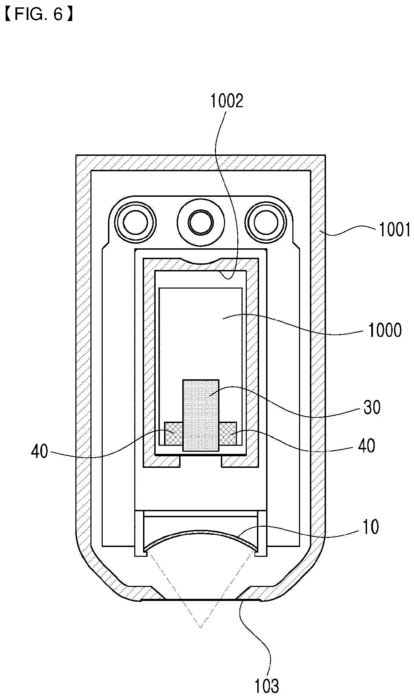

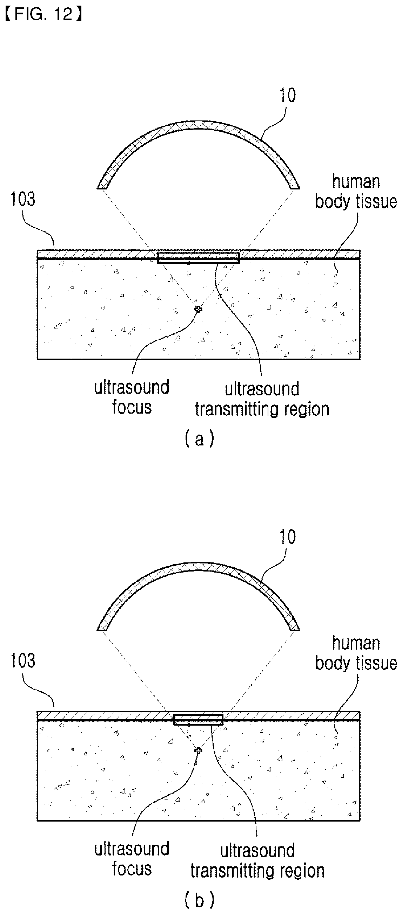

Such ultrasound irradiation information may be particularly useful when the position of the ultrasound transducer 10 changes. Referring to FIG. 12, when the ultrasound transducer 10 is located at the position indicated in (a), the ultrasound transmitting region and the depth of the ultrasound focus are determined according to a predetermined condition, and when the ultrasound transducer 10 moves away from the human body surface (in an upward direction in FIG. 6) to be located at the position indicated in (b), the ultrasound wave trajectory and the ultrasound focus determined in accordance with the geometrical characteristics of the ultrasound transducer 10 also move together, and thus the depth of the ultrasound focus from the surface of the human tissue is reduced and the size of the ultrasound transmitting region where the ultrasound passes through the surface of the human tissue is also reduced. In an embodiment of the present invention, it is configured to variably display the depth of the ultrasound focus and the ultrasound transmitting region on the surface of the human tissue that change in accordance with the movement of the ultrasound transducer 10. In this case, a sensor that detects the amount of change in the position of the ultrasound transducer 10 may be provided to detect, and the movement of the ultrasound transducer 10 may be detected using the signal of the sensor and the change in the focus may be calculated accordingly.

Although not described in detail, the above-described information may be displayed on the image display module 60.

The focused ultrasound device 100 may include a controller for controlling the operation of the motor, the power supply to the ultrasound transducer 10, the camera module 30, and the operation of the image display module 40. The controller may include necessary hardware and software and may be programmed to display the photographed human body surface image and the ultrasound irradiation information described above.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

### [Industrial Applicability]

The present invention relates to a focused ultrasound device, so it has an industrial applicability.

## Claims

1. A focused ultrasound device comprising:
an ultrasound transducer having a piezoelectric element for generating ultrasound waves;
a housing in which the ultrasound transducer is installed; and
a camera module configured to photograph a surface of a human body onto which the ultrasound waves generated by the ultrasound transducer are irradiated.

2. The focused ultrasound device of claim 1, wherein the camera module is disposed inside the housing or on the housing.

3. The focused ultrasound device of claim 1, wherein the camera module is disposed within the piezoelectric element.

4. The focused ultrasound device of claim 3, wherein the camera module is disposed at a center of the piezoelectric element.

5. The focused ultrasound device of claim 1, wherein the camera module is disposed outside the piezoelectric element.

6. The focused ultrasound device of claim 1, further comprising a lighting module for illuminating an image photographing region of the camera module.

7. The focused ultrasound device of claim 1, wherein the lighting module is disposed inside the housing or on the housing.

8. The focused ultrasound device of claim 7, wherein the lighting module is disposed near a periphery of the camera module.

9. The focused ultrasound device of claim 7, wherein the lighting module is disposed outside an outer periphery of the piezoelectric element.

10. The focused ultrasound device of claim 1, wherein the ultrasound transducer is provided with a through hole into which the camera module is installed, further comprising a tube member that is inserted into the through hole in a state of sealing the through hole, and wherein the camera module is disposed in the tube member.

11. The focused ultrasound device of claim 10, further comprising a lighting module for illuminating an image photographing region of the camera module, wherein the lighting module is disposed within the tube member together with the camera module.

12. The focused ultrasound device of claim 10, wherein a front portion of the tube member is formed of a transparent material and a rear end thereof is formed to be sealed.

13. The focused ultrasound device of claim 1, wherein the housing has an opening portion through which the ultrasound passes and an ultrasound transmitting film covering the opening portion, further comprising a lighting module that is installed within the housing and is configured to illuminate an image photographing region of the camera module, wherein the camera module is arranged to photograph through the ultrasound transmitting film and the lighting module is arranged to illuminate through the ultrasound transmitting film, and wherein the ultrasound transmitting film is formed of a transparent material or a partially transparent material to enable a photographing by the cameral module and an illumination by the lighting module.

14. The focused ultrasound device of claim 1, further comprising a support that is configured to be coupled to and decoupled from the housing, wherein the camera module is mounted to the support.

15. The focused ultrasound device of claim 14, wherein the housing is provided with an insertion hole for an insertion of the support, and wherein the support is inserted into the insertion hole.

16. The focused ultrasound device of claim 14, wherein the camera module is provided in plurality.

17. The focused ultrasound device of claim 14, further comprising a lighting module configured to illuminate a human body surface onto which the ultrasound is irradiated, wherein the lighting module is mounted to the support together with the camera module.

18. The focused ultrasound device of claim 17, wherein the lighting module is provided in plurality.

19. The focused ultrasound device of claim 1, wherein the cameral module is installed to the housing, and wherein the ultrasound transducer is formed as a part of an ultrasound transducer module that is configured to be separated from and be coupled to the housing.

20. The focused ultrasound device of claim 19, wherein the ultrasound transducer module comprises:
a support structure that supports the ultrasound transducer and is configured to be able to be coupled to the housing;
a hollow pipe that is installed to penetrate the support structure and the ultrasound transducer; and
a transparent cover that is coupled to a front end of the hollow pipe,
and wherein the camera module is configured such that a front end portion of the camera module is inserted into the hollow pipe in a state that the ultrasound transducer module is coupled to the housing.

21. The focused ultrasound device of claim 19, wherein the ultrasound transducer module comprises:
a support structure that supports the ultrasound transducer and is configured to be able to be coupled to the housing;
a through hole that penetrates the support structure and the ultrasound transducer; and
a transparent cover that is coupled to the through hole,
and wherein the camera module is inserted into the through hole in a direction toward the transparent cover in a state that the ultrasound transducer module is coupled to the housing.

22. The focused ultrasound device of claim 19, wherein the ultrasound transducer module comprises:
a support structure that supports the ultrasound transducer and is configured to be able to be coupled to the housing;
a hollow pipe that is installed to penetrate the support structure and the ultrasound transducer; and
a transparent cover that is coupled to a front end of the hollow pipe,
and wherein the camera module is installed in the hollow pipe toward the transparent cover in a state that the ultrasound transducer module is coupled to the housing.

23. The focused ultrasound device of one of claims 20 to 22, further comprising a lighting module that is configured to illuminate a human body surface on which the ultrasound is irradiated, wherein the lighting module is inserted into the hollow pipe or the through hole together with the camera module.

24. A focused ultrasound device comprising:
an ultrasound transducer having a piezoelectric element for generating ultrasound waves;
a housing in which the ultrasound transducer is installed;
a camera module configured to photograph a surface of a human body onto which the ultrasound waves generated by the ultrasound transducer are irradiated; and
an image display module displaying an image of a human body surface photographed by the camera module,
wherein the image display module is configured to display ultrasound irradiation information on the image of the surface of the human body photographed by the camera module.

25. The focused ultrasound device of claim 24, wherein the ultrasound irradiation information comprises at least one of an ultrasound transmitting region penetrating a human body surface, a position of an ultrasound focus formed within a human tissue, a depth from a human body surface to the ultrasound focus, an entire region on which the ultrasound irradiates while the ultrasound transducer moves, an entire region on which the ultrasound focus is located within the human tissue while the ultrasound transducer moves, an overall length of a trajectory of the ultrasound focus, and an interval between the ultrasound foci.

26. The focused ultrasound device of claim 25, wherein the housing comprises an opening portion through which the ultrasound passes and an ultrasound transmitting film that covers the opening portion, and wherein the ultrasound transmitting region is displayed to respond to a region in which the ultrasound generated by the ultrasound transducer passes through the ultrasound transmitting film.

27. The focused ultrasound device of claim 24, wherein the ultrasound transducer is formed as a plurality of replaceable cartridges, and the ultrasound irradiation information is obtained based on an ultrasound focusing shape of the ultrasound transducer.

28. The focused ultrasound device of claim 24, wherein the ultrasound transducer is installed to be movable, rotatable and tiltable, and wherein the image display module is configured to variably display the ultrasound irradiation information in accordance with the ultrasound irradiation information that changes in accordance with movement, rotation or tilting of the ultrasound transducer.
